# EUROPÄISCHE PATENTSCHRIFT

(11) **EP 0 568 085 B1**
(45) Veröffentlichungstag und Bekanntmachung des Hinweises auf die Patenterteilung: **27.11.1996**
(21) Anmeldenummer: 93107014.8
(22) Anmeldetag: 29.04.1993
(51) Int. Cl.: A61F 13/15

(54) **Wegwerfwindel mit hautberührender Decklage mit einer elastischen Öffnung sowie Verfahren zur Herstellung der elastischen Öffnung**
Disposable diaper with a skin touching top-sheet with an elastic opening and process for the production of the elastic opening
Couche à jeter avec feuille supérieur touchant la peau avec une ouverture élastique et procédé pour la fabrication de l'ouverture élastique

(30) Priorität: 30.04.1992 JP 111859/92
(43) Veröffentlichungstag der Anmeldung: 03.11.1993
(73) Patentinhaber: UNI-CHARM CORPORATION, Kawanoe-shi Ehime-ken (JP)
(72) Erfinder: Tanji, Hiroyuki, Kawanoe-shi, Ehime-ken (JP); Wada, Ichiro, Kawanoe-shi, Ehime-ken (JP); Ono, Yoshio, Kawanoe-shi, Ehime-ken (JP); Soga, Hiroyuki, Kawanoe-shi, Ehime-ken (JP)
(74) Vertreter: Sperling, Rüdiger, Dipl.-Ing.

(56) Entgegenhaltungen:
- EP-A- 0 403 832
- WO-A-83/03051
- WO-A-91/08717
- FR-A- 2 644 694

## Beschreibung

Diese Erfindung betrifft eine Wegwerfwindel mit einer hautberührenden Decklage, die mit einer elastischen Öffnung zum zuverlässigen Einführen von Ausscheidungen versehen ist und ebenso ein Verfahren zur Herstellung dieser elastischen Öffnung.

Die japanische Gebrauchsmusteranmeldung Amtsblatt-Nr. 1974-120439 zeigt eine Windeleinlage mit einer Decklage auf, in deren mittlerem Bereich eine Öffnung ausgebildet ist, die sich in Längsrichtung weiter als in Querrichtung der Decklage erstreckt, wobei die Öffnung entlang ihrem Umfangsrand mit einem in Längsrichtung dehnbaren elastischen Element versehen ist, um so eine elastische Linie in Form eines geschlossenen Ringes zu bilden. Die japanische Patentanmeldung Amtsblatt-Nr. 1986-41304 Zeigt ebenfalls eine Wegwerfwindel mit einer Decklage auf, die in ihrem Mittelbereich mit einer Öffnung versehen ist, die sich in Längsrichtung weiter als in Querrichtung der Decklage erstreckt, wobei die Öffnung entlang ihren seitlich einander gegenüberliegenden Seitenrändern jeweils mit elastischen Elementen versehen ist. Eine ähnliche Windel wird in der EP-A-0 566 150 gezeigt und beschrieben.

Die vorstehend genannte japanische Gebrauchsmusteranmeldung Amtsblatt-Nr. 1974-120439 und die japanische Patentanmeldung Amtsblatt-Nr. 1986-41304 zeigen beide weder ein Verfahren zur Ausbildung der Öffnung in der hautberührenden Decklage noch ein Verfahren zum Anbringen des elastischen Elementes um die Öffnung auf. Es wird angenommen, daß das elastische Element in endloser Ausführung entlang dem gesamten Umfang der Öffnung unmittelbar am Umfangsrand befestigt wird und anschließend das elastische Element mit dem Umfangsrand der Öffnung überdeckt wird. Dafür findet sich in der Beschreibung jedoch kein spezielles Verfahren. Angesichts der Tatsache, daß das erstgenannte Dokument eine wiederverwendbare Windeleinlage betrifft, bedient sich das Verfahren vermutlich Schneid- und Nähtechniken bekannter Art. Gemäß dem zweiten Dokument wird der Mittelbereich der Lage ausgeschnitten, um die Öffnung zu bilden, und die beiden Seitenränder der Öffnung werden mit den jeweiligen elastischen Elementen versehen, die an den Rückseiten der jeweiligen Seitenränder unter Verwendung von Klebstoff angebracht werden. Folglich ist die Windel gemäß dem zweiten Dokument nicht nur unansehnlich, sondern auch hinsichtlich der gewünschten Festigkeit entlang dem Umfangsrand der Öffnung in nicht akzeptabler Weise mangelhaft, da die elastischen Elemente in keiner Weise bedeckt sind, obwohl sie an der Rückseite der Lage angeordnet sind. Darüber hinaus führt eine derartige Anordnung nicht zur Bildung von ordentlich gleichmäßigen Raffungsfältchen entlang dem gesamten Umfangsrand der Öffnung, so daß daher nicht erwartet werden kann, daß der gesamte Umfangsrand der Öffnung dicht an der Haut des Trägers anliegt.

Sowohl bei der Windeleinlage als auch bei der Windel, die in der vorstehend genannten japanischen Gebrauchsmusteranmeldung Amtsblatt-Nr. 1974-120439 bzw. der japanischen Patentanmeldung Amtsblatt-Nr. 1986-41304 aufgezeigt sind, wird die Öffnung durch das an ihr angebrachte elastische Element mit elastischen Eigenschaften versehen, so daß der Umfang derselben dazu neigt, sich von einer getrennt vorgesehenen Decklage, die unter der hautberührenden Decklage liegt, abzuheben. Die hautberührende Decklage ist jedoch nur am Umfang der Öffnung mit dem elastischen Element versehen, so daß folglich ein erwünschter Grad eines derartigen Anhebeeffektes nicht aus dieser bekannten Anordnung erwartet werden kann. Mit anderen Worten kann kein angemessener Abstand zwischen der hautberührenden Decklage, in deren Mitte die Öffnung ausgebildet ist, und der getrennt vorgesehenen Decklage, die unter der hautberührenden Decklage liegt, erzielt werden. Demgemäß kann dieser Raum oftmals nicht als Tasche dienen, in die die Ausscheidungen zuverlässig eingeführt werden sollten, und die Haut des Trägers kann in unerwünschter Weise mit Ausscheidungen verschmiert werden. Eine derartige Unannehmlichkeit wird insbesondere dann auftreten, wenn die Windel dicht am Körper des Trägers getragen wird, wobei die hautberührende Decklage gegen den Schrittbereich des Trägers gepreßt wird.

Im Hinblick auf diese Probleme ist es eine wesentliche Aufgabe der Erfindung, eine neuartige Anordnung der Wegwerfwindel aufzuzeigen, die mit elastischen Elementen versehen ist, die sich nicht nur entlang den einander seitlich gegenüberliegenden Seitenrändern der Öffnung erstrecken, sondern auch weiter über die in Längsrichtung einander gegenüberliegenden Enden der Öffnung hinaus im wesentlichen zu in Längsrichtung einander gegenüberliegenden Enden der hautberührenden Decklage, so daß die hautberührende Decklage, in deren Mittelbereich die Öffnung ausgebildet ist, sich von der getrennt vorgesehenen Decklage, die unterhalb der hautberührenden Decklage liegt, in einem Maß erheben kann, das ausreicht, um einen gewünschten Taschenraum zu bilden.

### BESCHREIBUNG DER ERFINDUNG

Im Hinblick auf die vorstehend dargelegte Aufgabe wird erfindungsgemäß zunächst eine Wegwerfwindel mit einer hautberührenden Decklage aufgezeigt, die mit einer elastischen Öffnung versehen ist, wobei die Windel eine flüssigkeitsdurchlässige erste Decklage, eine flüssigkeitsundurchlässige Außenlage, einen flüssigkeitsabsorbierenden Kern, der zwischen die erste Decklage und die Außenlage gelegt ist, und eine flüssigkeitsbeständige zweite Decklage aufweist, die über der ersten Decklage liegt, um die hautberührende Decklage zu bilden, die zweite Decklage im wesentlichen in ihrem Mittelbereich mit der in Längsrichtung weiter als in Querrichtung der zweiten Decklage sich erstreckenden Öffnung versehen ist, die zweite Decklage entlang ihrem äußeren Umfang mit der ersten Decklage verbunden ist und in Längsrichtung elastisch dehnbare Elemente entlang den einander gegenüberliegenden Seitenrändern der Öffnung an der zweiten Decklage angebracht sind, dadurch gekennzeichnet, daß die elastischen Elemente erste und zweite elastische Elemente umfassen, die unabhängig voneinander in der Weise vorgesehen sind, daß sie entlang den einander gegenüberliegenden Seitenrändern der Öffnung sich erstrecken und im wesentlichen an den in Längsrichtung gegenüberliegenden Enden der zweiten Decklage enden.

Vorzugsweise umfaßt die zweite Decklage erste und zweite Lagenelemente.

Vorzugsweise sind die ersten und zweiten Lagenelemente entlang beidseits einander gegenüberliegenden Innenrändern in der Nähe der in Längsrichtung einander gegenüberliegenden Enden der ersten und zweiten Lagenelemente miteinander verbunden.

Vorzugsweise überschneiden oder berühren die ersten und zweiten elastischen Elemente einander an in Längsrichtung gegenüberliegenden Enden der Öffnung.

Die Erfindung zeigt ebenso ein Verfahren zur Bildung der hautberührenden Decklage einer derartigen neuartigen Windel mit der elastischen Öffnung auf, umfassend die Schritte:
A. Zuführen von ersten und zweiten elastischen Elementen, die beide gedehnt gehalten werden, auf eine erste Endlosbahn in Längsrichtung derselben, so daß die ersten und zweiten endlosen elastischen Elemente sich auf dem Mittelbereich der ersten Endlosbahn erstrecken, der von einander in Querrichtung gegenüberliegenden ersten und zweiten Seitenrändern beabstandet verläuft, die ersten und zweiten endlosen elastischen Elemente zueinander in Querrichtung der ersten endlosen Bahn beabstandet sind, wobei sie wechselweise Wellenkämme und relativ breite Wellentäler beschreiben, und wobei die jeweiligen Wellenkämme, die vom ersten elastischen Element beschrieben werden, Mittelbereichen der jeweiligen Täler, die vom zweiten endlosen elastischen Element beschrieben werden, gegenüberliegend angeordnet sind, und die jeweiligen Wellenkämme, die vom zweiten endlosen elastischen Element beschrieben werden, Mittelbereichen der jeweiligen Wellentäler gegenüberliegend angeordnet sind, die vom ersten endlosen elastischen Element beschrieben werden, und gleichzeitiges Verkleben der ersten und zweiten elastischen Elemente gemeinsam unter Verwendung von Klebstoff zur Bildung einer ersten endlosen Verbundbahn;
B. Verbinden einer zweiten endlosen Bahn mit der ersten endlosen Verbundbahn in dem Gebiet derselben, innerhalb welchem die ersten und zweiten endlosen elastischen Elemente an der ersten endlosen Verbundbahn befestigt sind, um eine zweite endlose Verbundbahn zu bilden;
C. Ausschneiden des Teils der zweiten endlosen Verbundbahn, der zwischen dem ersten und zweiten endlosen elastischen Element, die sich parallel zueinander und voneinander in Querrichtung der zweiten endlosen Verbundbahn beabstandet entlang den jeweiligen Wellenkämmen und Wellentälern erstrecken, bestimmt ist, um so Ausschnitte zu bilden, die zur Bildung der Öffnung bestimmt sind und dadurch dritte endlose Verbundbahnelemente (A) und (B) zu bilden;
D. Verschieben der dritten endlosen Verbundbahnelemente (A) und (B) in Längsrichtung relativ zueinander, so daß die jeweiligen Wellenkämme der dritten endlosen Verbundbahnelemente (A) und (B) einander symmetrisch in überlappender Beziehung in Querrichtung dieser dritten endlosen Verbundbahnelemente (A) und (B) gegenüberliegen und die jeweiligen Wellentäler derselben ebenfalls einander symmetrisch gegenüberliegend angeordnet sind, aber zwischen sich die jeweiligen Öffnungen bilden; und
E. Schneiden der dritten endlosen Verbundbahnelemente (A) und (B) in Querrichtung derselben entlang gedachten Linien, die vertikal die jeweiligen Paare von beidseits einander gegenüberliegend angeordneten Wellenkämme in Hälften teilen.

Vorzugsweise schließt der Schritt D einen Schritt des Verbindens der dritten endlosen Verbundbahnelemente (A) und (B) in überlappender Beziehung zueinander entlang inneren Seitenrändern der jeweiligen Paare von beidseits einander gegenüberliegend angeordneten Wellenkämmen ein.

Unter der Zugkraft der elastischen Elemente, die sich entlang den einander gegenüberliegenden Seitenrändern der Öffnung und weiter über die in Längsrichtung gegenüberliegenden Enden der Öffnung hinaus im wesentlichen bis zu den in Längsrichtung gegenüberliegenden Enden der zweiten Decklage (d.h. der hautberührenden Decklage) erstrecken, wird die zweite Decklage von der unter ihr angeordneten Decklage abgehoben, was um die Öffnung und die Abschnitte der elastischen Elemente, die sich zwischen den in Längsrichtung gegenüberliegenden Enden der Öffnung und den in Längsrichtung gegenüberliegenden Enden der zweiten Decklage erstrecken, im stärksten Maße auftritt, wodurch verhindert wird, daß sich die Öffnung seitlich verschiebt, da dieser Abschnitt der elastischen Elemente notwendigerweise entlang der Mittellinie des Schrittbereiches zu positionieren ist, soweit die Windel ordnungsgemäß am Körper des Trägers getragen wird.

### KURZE BESCHREIBUNG DER ZEICHNUNG

Die Erfindung wird anhand eines Beispiels unter Bezug auf die beiliegenden Figuren beschrieben, wobei:
- Fig. 1: eine perspektivische Ansicht einer Innenseite einer Wegwerfwindel mit einer hautberührenden Decklage gemäß der Erfindung zeigt;
- Fig. 2: eine Schnittdarstellung entlang einer Linie 2-2 in Fig. 1 zeigt;
- Fig. 3: eine schematische Draufsicht zur Erläuterung der Weise zeigt, in der erste und zweite elastische Elemente auf einer Endlosbahn angebracht werden, die als Ausgangsmaterial für eine zweite Decklage (d.h. die hautberührende Decklage) verwendet wird, um die erste endlose Verbundbahn zu bilden;
- Fig. 4: eine schematische Draufsicht zur Erläuterung der Weise zeigt, in der eine zweite endlose Bahn mit der ersten endlosen Verbundbahn in dem Bereich, innerhalb dessen die ersten und zweiten elastischen Elemente angebracht sind, verbunden wird, um so eine zweite endlose Verbundbahn zu bilden;
- Fig. 5: eine schematische Draufsicht zur Erläuterung der Weise zeigt, in der der Teil der zweiten endlosen Verbundbahn, der zwischen den ersten und zweiten elastischen Elementen gebildet ist, ausgeschnitten wird, um dritte endlose Verbundbahnelemente (A) und (B) zu bilden; und
- Fig. 6: eine schematische Draufsicht zur Erläuterung der Weise zeigt, in der die dritten endlosen Verbundbahnelemente (A) und (B) in Längsrichtung relativ zueinander verschoben werden, so daß zwischen diesen Öffnungen gebildet werden.

Zunächst wird die gemäß der Erfindung aufgebaute Windel anhand eines Beispiels unter Bezug auf die Figuren erläutert.

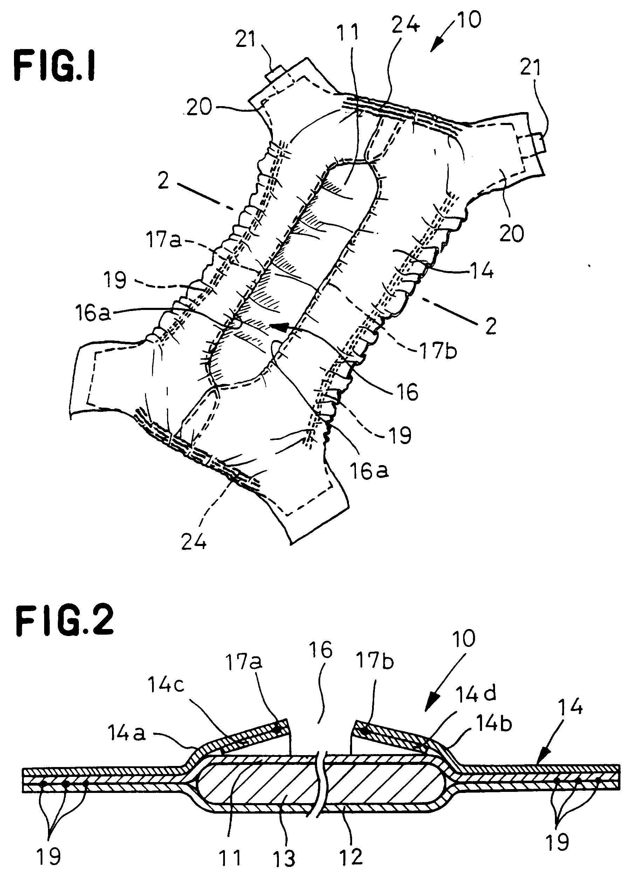

Wie Fig. 1 und 2 zeigen, umfaßt eine Windel 10 eine flüssigkeitsdurchlässige erste Decklage 11, eine flüssigkeitsundurchlässige Außenlage 12, einen zwischen diese gelegten flüssigkeitsabsorbierenden Kern 13 und eine flüssigkeitsbeständige zweite Decklage 14, die über der ersten Decklage 11 liegt. Im Mittelbereich der zweiten Decklage 14 ist eine Öffnung 16 ausgebildet, die in Längsrichtung länger ist als in Querrichtung der Lage 14 und deren in Längsrichtung gegenüberliegende Enden jeweils Kreisbögen beschreiben. Die Öffnung 16 kann wenigstens innerhalb des Schrittbereiches ausgebildet sein.

Die zweite Decklage 14 umfaßt eigentlich zwei Lagenelemente 14a, 14b, die seitlich einander überlappen und beidseits gegenüberliegend angeordnete innere Seitenränder jeweils aufweisen, die mit Ausschnitten 16a versehen sind, so daß jeweils zwei beidseits einander gegenüberliegend angeordnete Ausschnitte die Öffnung 16 bilden. Verstärkungslagenelemente 14c, 14d, die aus demselben Material hergestellt werden, wie für die Lagenelemente 14a, 14b verwendet, werden mit den Rückseiten der jeweiligen Ausschnitte 16a entlang den Rändern derselben verbunden. Weiter sind in Längsrichtung dehnbare erste und zweite elastische Elemente 17a, 17b vorgesehen, die jeweils mehrere elastische Fäden umfassen und in gedehntem Zustand zwischen den Lagenelementen 14a und 14b und den Verstärkungslagenelementen 14c und 14d entlang den Rändern der jeweiligen Ausschnitte 16a unter Verwendung von Heißschmelzklebers (nicht dargestellt) angebracht werden. Diese ersten und zweiten elastischen Elemente 17a, 17b erstrecken sich weiter über die in Längsrichtung gegenüberliegenden Enden der Öffnung 16 hinaus im wesentlichen zu den Längsrichtung einander gegenüberliegend angeordneten Enden der Lagenelemente 14a, 14b. Die beidseits einander gegenüberliegend angeordneten Innenränder der jeweiligen Lagenelemente 14a, 14b sind in der Nähe der jeweiligen in Längsrichtung gegenüberliegenden Enden der Lagenelemente überlappt und unter Verwendung von Heißschmelzkleber (nicht dargstellt) miteinander verklebt.

Die ersten und zweiten elastischen Elemente 17a,17b sind unabhängig voneinander angeordnet, im Gegensatz zu dem einzelnen endlosen elastischen Element, das einen endlosen Ring bildet, wie es herkömmlicherweise in einer bekannten Windeleinlage verwendet wird, und zusätzlich sind die beidseits einander gegenüberliegenden inneren Seitenränder der jeweiligen Lagenelemente 14a, 14b, die in der Nähe der in Längsrichtung gegenüberliegenden Enden derselben einander überlappen, unter Verwendung von Heißschmelzkleber miteinander verbunden. Eine derartige einzigartige Anordnung erlaubt es, die zweite Decklage 14 ohne weiteres entlang den überlappenden inneren Seitenrändern abzureißen, wenn es erwünscht ist, beinahe die gesamte Fläche der ersten Decklage 11, die unter der zweiten Decklage 14 liegt, freizulegen und dadurch feste Ausscheidungen, die zwischen der ersten Decklage 11 und zweiten Decklage 14 gehalten werden, abzukratzen. Auch wenn die überlappenden inneren Seitenränder unter Verwendung des Klebers miteinander verbunden werden, hat der für das Zusammensetzen einer derartigen Windel verwendete Heißschmelzkleber im allgemeinen auch nach dem Aushärten keine ausreichende Klebekraft, um den Abreißvorgang zu erschweren.

Flüssige Ausscheidungen werden durch die Öffnung 16 auf die erste Decklage 11 geführt und vom Kern 13 absorbiert, während feste Ausscheidungen in einen Taschenraum eingeführt werden, der zwischen der ersten Decklage 11 und der zweiten Decklage 14 gebildet ist. Derartige feste Ausscheidungen können von der ersten Decklage 11, die in einem gewünschten Ausmaß freigelegt ist, wenn die zweite Decklage 14 von den in Längsrichtung gegenüberliegenden Enden der Öffnung 16 abgerissen wurde, abgekratzt werden.

Zwischen seitlich einander gegenüberliegenden Rändern der Decklage 11 und seitlich gegenüberliegenden Rändern der Außenlage 12, die beide über die beiden Seiten des flüssigkeitsabsorbierenden Kerns 13 sich nach außen erstrecken, sind mehrere elastische Elemente 19, die jeweils wiederum mehrere elastische Fäden umfassen, in gedehntem Zustand unter Verwendung von Heißschmelzkleber (nicht dargestellt) jeweils angebracht, so daß sie in Längsrichtung der Lagen dehnbar sind und dicht um die Beine des Trägers anliegen. Ähnlich sind zwischen den in Längsrichtung gegenüberliegenden Enden der Decklage 11 und den zugehörigen Enden der Außenlage 12 mehrere elastische Elemente 24 vorgesehen, die jeweils wiederum mehrere elastische Fäden umfassen, so daß sie quer zu den Lagen dehnbar sind und dicht um die Hüfte des Trägers anliegen.

Die Decklage 11 kann aus Vliesstoff, poröser Kunststoffolie oder ähnlichem, die Außenlage 12 aus Kunststoffolie, einer laminierten Lage aus dieser Kunststoffolie und Vliesstoff oder ähnlichem und der flüssigkeitsabsorbierende Kern aus einer Mischung von Faserpulpe und hochabsorbierendem Polymerpulver oder ähnlichem hergestellt sein. Die hautberührende Decklage 14 und die Verstärkungslagenelemente 14c, 14d sind vorzugsweise aus wasserabstoßendem und hoch luftdurchlässigem Vliesstoff hergestellt. Es versteht sich, daß in der Beschreibung der Erfindung der Begriff "flüssigkeitsbeständig" sich auf Material bezieht, das einen ausreichenden Grad von wasserabstoßenden Eigenschaften hat, um das problemlose Durchdringen von flüssigen Ausscheidungen zu verhindern, wenn die Windel am Körper des Trägers getragen wird.

Wie Fig. 1 zeigt, hat die Windel 10 zwei paarweise angeordnete flügelähnliche Klappen 20, die sich von den seitlich gegenüberliegend angeordneten Seiten der Hüftlinie jeweils nach außen erstrecken. Freie Enden von Befestigungsbändern 21, die an den jeweiligen Rückseiten der flügelähnlichen Klappen 20 angebracht sind, können durch Verkleben mit der Außenlage 12 an der Vorderseite befestigt werden, um die Windel 10 um den Körper des Trägers aufzurichten.

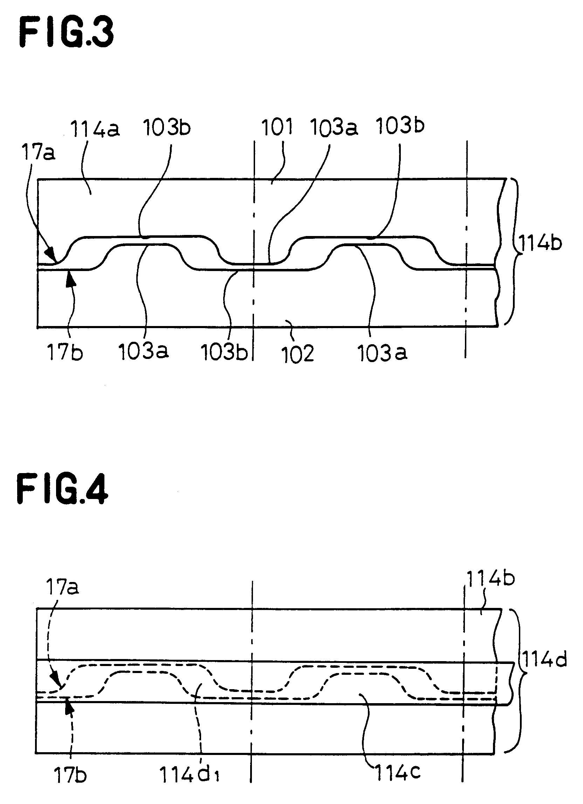

Nachfolgend wird anhand eines Beispiels unter Bezug auf Fig. 3 bis 6 ein erfindungsgemäßes Verfahren beschrieben.

Wie Fig. 3 zeigt, werden ein erstes und zweites elastisches Element 17a, 17b, die beide gedehnt gehalten werden, von einer Traversiereinrichtung (nicht dargestellt) auf eine erste Endlosbahn 114a in Längsrichtung derselben zugeführt, so daß das erste und zweite endlose elastische Element 17a, 17b sich im Mittelbereich der ersten Endlosbahn 114a erstreckt, der von den einander seitlich gegenüberliegenden ersten und zweiten Rändern 101, 102 der ersten Endlosbahn 114a beabstandet ist, wobei das erste und zweite endlose elastische Element 17a, 17b voneinander in Querrichtung der ersten Endlosbahn 114a beabstandet sind und wechselweise Wellenkämme 103a und relativ weite Wellentäler 103 jeweils beschreiben, wobei die jeweiligen Wellenkämme 103a, die vom ersten endlosen elastischen Element 17a beschrieben werden, den Mittelabschnitten der jeweiligen Wellentäler 103b gegenüberliegen, die vom zweiten endlosen elastischen Element 17b beschrieben werden, während die jeweiligen Kämme 103a, die vom zweiten endlosen elastischen Element 17b beschrieben werden, Mittelabschnitten der jeweiligen Täler 103b gegenüberliegen, die vom ersten endlosen elastischen Element 17a beschrieben werden, und das erste und zweite elastische Element 17a, 17b gemeinsam unter Verwendung von Heißschmelzkleber (nicht dargestellt) verklebt werden, um eine erste endlose Verbundbahn 114b zu bilden.

Wie Fig. 4 zeigt, wird eine zweite endlose Bahn 114c, die schmäler ist als die erste endlose Verbundbahn 114b, mit der ersten endlosen Verbundbahn 114b unter Verwendung von Heißschmelzkleber (nicht dargestellt) entlang dem Bereich derselben, innerhalb dessen das erste und zweite endlose elastische Element 17a, 17b an der ersten endlosen Verbundbahn 114b befestigt sind, verbunden, um eine zweite endlose Verbundbahn 114d zu bilden.

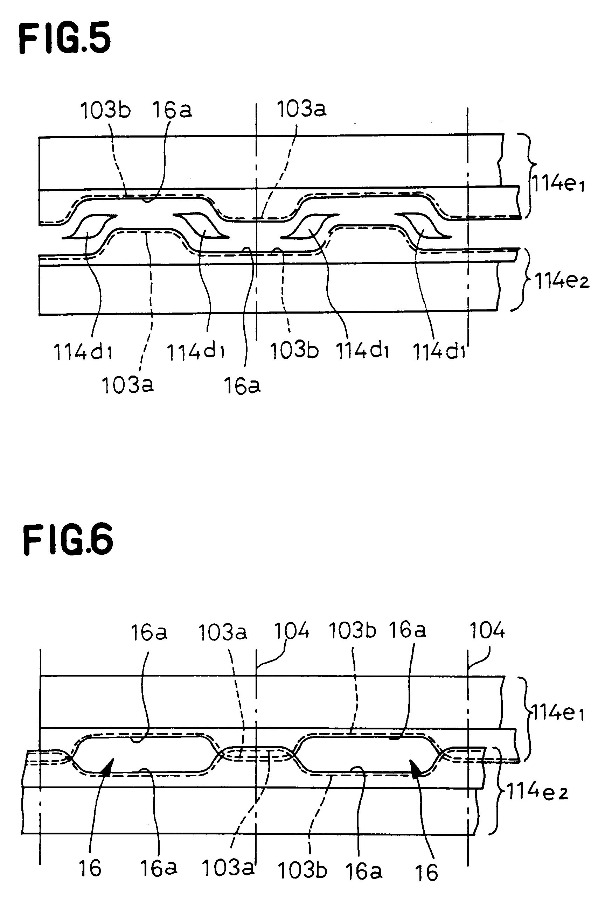

Wie in Fig. 4 und 5 gezeigt, wird der Teil 114d₁ der zweiten endlosen Verbundbahn 114d, der zwischen dem ersten endlosen elastischen Element 17a und dem zweiten endlosen elastischen Element 17b bestimmt ist, die sich parallel zueinander und in Querrichtung der zweiten endlosen Verbundbahn 114d voneinander beabstandet erstrecken, innerhalb der Kämme 103a und der Täler 103b ausgeschnitten, um so Ausschnitte 16a zu bilden, die zur Bildung der Öffnungen 16 bestimmt sind, und dadurch dritte endlose Verbundbahnelemente (A) und (B) 114e₁, 114e₂ zu bilden.

Wie Fig. 6 zeigt, werden die dritten endlosen Verbundbahnelemente (A) und (B) 114e₁, 114e₂ in Längsrichtung relativ zueinander verschoben, so daß die jeweiligen Wellenkämme 103a der dritten endlosen Verbundbahnelemente (A) und (B) 114e₁, 114e₂ einander symmetrisch in überlappender Beziehung in QUerrichtung der dritten endlosen Verbundbahnelemente (A) und (B) 114e₁, 114e₂ gegenüberliegen und die jeweiligen Wellentäler 103b (oder die Ausschnitte 16a) ebenso symmetrisch einander gegenüberliegen, jedoch zwischen sich die jeweiligen Öffnungen 16 bilden. Anschließend werden die dritten endlosen elastischen Verbundbahnelemente (A) und (B) 114e₁, 114e₂ in überlappender Beziehung unter Verwendung von Heißschmelzkleber (nicht dargestellt) entlang den inneren Seitenrändern der jeweiligen Paare von einander beidseits gegenüberliegenden Wellenkämmen 103a verbunden. Es versteht sich, daß diese inneren Seitenränder auf Wunsch auch unverklebt belassen werden können.

Die dritten endlosen Verbundbahnelemente (A) und (B) 114e₁, 114e₂, werden entlang gedachten Linien 104, die vertikal die jeweiligen Paare von beidseits einander gegenüberliegend angeordneten Wellenkämmen 103 halbieren, in Querrichtung geschnitten. Dieser Schneidschritt kann durchgeführt werden, wenn die dritten endlosen elastischen Verbundbahnelemente (A) und (B) 114e₁, 114e₂ auf der obersten Lage der endlosen Windelbahn angeordnet sind, die mehrere einzelne Windeln, wie in Fig. 1 und 2 dargestellt (mit Ausnahme des Fehlens der zweiten Decklage 14), umfaßt, um die einzelnen Windeln 10 aus Fig. 1 und 2 zu erhalten.

Das Verfahren zur Herstellung einer derartigen endlosen Windelbahn, die eine Vielzahl von einzelnen Windeln 10 umfaßt, wie in Fig. 1 (mit Ausnahme des Nichtvorhandenseins der zweiten Decklage 14) dargestellt, die aufeinanderfolgend endlos miteinander in Längsrichtung derselben vorliegen, ist dem Fachmann aus verschiedenen Patentbeschreibungen bekannt, so daß daher die Einzelheiten eines derartigen Verfahrens hier nicht beschrieben werden.

Während die Ausführungsform unter Bezug auf sogenannte offene Windeln erläutert und beschrieben wurde, die zum Schließen der Hüftlinie um die Hüfte des Trägers Befestigungsbänder verwenden, ist die Erfindung auch auf sogenannte Windeln des Höschentyps (einschließlich Erziehungshöschen) anwendbar, die eine endlose Hüftlinie aufweisen.

Wie aus vorstehender Beschreibung ohne weiteres verständlich ist, ermöglicht bei der erfindungsgemäßen Windel die Kontraktionskraft der elastischen Elemente, die sich entlang den einander gegenüberliegenden Seitenrändern der Öffnung erstrecken und sich weiter über die in Längsrichtung gegenüberliegenden Enden der Öffnung hinaus im wesentlichen zu den in Längsrichtung gegenüberliegenden Enden der zweiten Decklage (d.h. der hautberührenden Decklage) erstrecken, das Abheben der zweiten Decklage von der ersten Decklage, die unter der zweiten Decklage angeordnet ist, im bedeutendsten Ausmaß um die Öffnung, wodurch sie es ermöglicht, daß die erste und zweite Decklage zwischen sich einen Taschenraum bilden, der ausreicht, um ein zuverlässiges Einführen von Ausscheidungen sicherzustellen. Darüber hinaus dienen die Abschnitte der elastischen Elemente, die sich zwischen den jeweiligen in Längsrichtung gegenüberliegenden Enden erstrecken, dazu, zu verhindern, daß die Öffnung sich seitlich verschiebt, da diese Abschnitte der elastischen Elemente notwendigerweise entlang der Mittellinie des Schrittbereiches positioniert sein sollten, soweit die Windel in geeigneter Weise am Körper des Trägers getragen wird, und somit fließen Ausscheidungen zuverlässig durch die Öffnung in den Taschenraum.

Zwei Lagenelemente, die die jeweiligen Innenränder der Öffnung aufweisen, entlang welchen das erste und das zweite elastische Element sich unabhängig voneinander erstrecken und befestigt sind, werden seitlich nebeneinander in symmetrischer Beziehung angeordnet, um die zweite Decklage zu bilden, so daß die beidseits einander gegenüberliegenden Seitenränder der jeweiligen Lagenelemente, die sich von den jeweiligen in Längsrichtung gegenüberliegenden Enden der Öffnung zu den jeweiligen in Längsrichtung gegenüberliegenden Enden der zweiten Decklage erstrecken, leicht voneinander getrennt werden können. Die zweite Decklage kann leicht abgerissen werden, um so die erste Decklage unter der zweiten Decklage in einem gewünschten Ausmaß freizulegen und dadurch feste Ausscheidungen, die auf der ersten Decklage zurückgehalten sind, in effektiver Weise abkratzen zu können.

Das Verfahren zur Bildung der elastischen Öffnung gemaß der Erfindung erlaubt es, die ersten und zweiten elastischen Elemente, die am Umfangsrand der Öffnung angebracht sind, mit demselben Material zu bedecken, das für die zweite Decklage verwendet wird, wodurch die Bildung der ordentlich ausgeführten elastischen Öffnung erleichtert ist.

## Patentansprüche

1. Wegwerfwindel mit einer hautberührenden Decklage, die mit einer elastischen Öffnung (16) versehen ist, wobei die Windel (10) eine flüssigkeitsdurchlässige erste Decklage (11), eine flüssigkeitsundurchlässige Außenlage (12), einen flüssigkeitsabsorbierenden Kern (13), der zwischen die erste Decklage (11) und die Außenlage (12) gelegt ist, und eine flüssigkeitsbeständige zweite Decklage (14) aufweist, die über der ersten Decklage (11) liegt, um die hautberührende Decklage zu bilden, die zweite Decklage (14) im wesentlichen in ihrem Mittelbereich mit der in Längsrichtung weiter als in Querrichtung der zweiten Decklage (14) sich erstreckenden Öffnung (16) versehen ist, die zweite Decklage (14) entlang ihrem äußeren Umfang mit der ersten Decklage (11) verbunden ist und in Längsrichtung elastisch dehnbare Elemente (17a,17b) entlang den einander gegenüberliegenden Seitenrändern der Öffnung (16) an der zweiten Decklage (14) angebracht sind, wobei die elastischen Elemente (17a,17b) erste und zweite elastische Elemente (17a,17b) umfassen, die unabhängig voneinander in der Weise vorgesehen sind, daß sie sich entlang den einander gegenüberliegenden Seitenrändern der Öffnung (16) erstrecken, wobei die ersten und zweiten elastischen Elemente (17a,17b) über die in Längsrichtung gegenüberliegenden Enden der Öffnung (16) hinaus an den Seitenrändern der zweiten Decklage (14) angeordnet sind und im wesentlichen an den in Längsrichtung gegenüberliegenden Enden der zweiten Decklage (14) enden.

2. Windel nach Anspruch 1, wobei die zweite Decklage (14) erste und zweite Lagenelemente (14a,14b) umfaßt.

3. Windel nach Anspruch 1, wobei die ersten und zweiten Lagenelemente (14a,14b) miteinander entlang beidseits einander gegenüberliegenden inneren Seitenrändern in der Nähe von in Längsrichtung gegenüberliegenden Enden der ersten und zweiten Lagenelemente (14a,14b) verbunden werden.

4. Windel nach Anspruch 1, wobei die ersten und zweiten elastischen Elemente (17a,17b) einander an den in Längsrichtung gegenüberliegenden Enden der Öffnung (16) überschneiden oder berühren.

5. Verfahren zur Bildung einer hautberührenden Decklage einer Wegwerfwindel mit einer elastischen Öffnung (16) mit folgenden Schritten:
A. Zuführen von ersten und zweiten elastischen Elementen (17a,17b), die beide gedehnt gehalten werden, auf eine erste Endlosbahn (114a) in Längsrichtung derselben, so daß sich die ersten und zweiten endlosen elastischen Elemente (17a,17b) auf dem Mittelbereich der ersten Endlosbahn (114a) erstrecken, der von einander in Querrichtung gegenüberliegenden ersten und zweiten Seitenrändern beabstandet verläuft, die ersten und zweiten endlosen elastischen Elemente (17a,17b) zueinander in Querrichtung der ersten endlosen Bahn (114a) beabstandet sind, wobei sie wechselweise Wellenkämme (103a) und relativ breite Wellentäler (103b) beschreiben, und wobei die jeweiligen Wellenkämme (103a), die vom ersten elastischen Element (17a) beschrieben werden, Mittelbereichen der jeweiligen Wellentäler (103b), die vom zweiten endlosen elastischen Element (17b) beschrieben werden, gegenüberliegend angeordnet sind, und die jeweiligen Wellenkämme (103a), die vom zweiten endlosen elastischen Element (17b) beschrieben werden, Mittelbereichen der jeweiligen Wellentäler (103b) gegenüberliegend angeordnet sind, die vom ersten endlosen elastischen Element (17a) beschrieben werden, und gleichzeitiges Verkleben der ersten und zweiten elastischen Elemente (17a,17b) unter Verwendung von Klebstoff zur Bildung einer ersten endlosen Verbundbahn (114b);
B. Verbinden einer zweiten endlosen Bahn (114c) mit der ersten endlosen Verbundbahn (114b) in dem Gebiet derselben, innerhalb welchem die ersten und zweiten endlosen elastischen Elemente (17a,17b) an der ersten endlosen Verbundbahn (114b) befestigt sind, um eine zweite endlose Verbundbahn (114d) zu bilden;
C. Ausschneiden des Teils (114d₁) der zweiten endlosen Verbundbahn (114d), der zwischen dem ersten endlosen elastischen Element (17a) und dem zweiten endlosen elastischen Element (17b), die sich parallel zueinander und voneinander in Querrichtung der zweiten endlosen Verbundbahn (114d) beabstandet entlang den jeweiligen Wellenkämmen (103a) und Wellentälern (103b) erstrecken, bestimmt ist, um so Ausschnitte (16a) zu bilden, die zur Begrenzung der Öffnung (16) bestimmt sind und dadurch dritte endlose Verbundbahnelemente (A) (114e₁) und (B) (114e₂) zu bilden;
D. Verschieben der dritten endlosen Verbundbahnelemente (A) (114e₁) und (B) (114e₂) in Längsrichtung relativ zueinander, so daß die jeweiligen Wellenkämme (103a) der dritten endlosen Verbundbahnelemente (A) (114e₁) und (B) (114e₂) einander symmetrisch in überlappender Beziehung in Querrichtung dieser dritten endlosen Verbundbahnelemente (A) (114e₁) und (B) (114e₂) gegenüberliegen und die jeweiligen Wellentäler (103b) derselben ebenfalls einander symmetrisch gegenüberliegend angeordnet sind, aber zwischen sich die jeweiligen Öffnungen (16) bilden; und
E. Schneiden der dritten endlosen Verbundbahnelemente (A) (114e₁) und (B) (114e₂) in Querrichtung derselben entlang gedachten Linien (104), die vertikal die jeweiligen Paare von beidseits gegenüberliegend angeordneten Wellenkämmen (103a) in Hälften teilen.

6. Verfahren nach Anspruch 5, wobei Schritt D einen Schritt des Verbindens der dritten endlosen Verbundbahnelemente (A) (114e₁) und (B) (114e₂) miteinander in überlappender Beziehung entlang inneren Seitenrändern der jeweiligen Paare von beidseits gegenüberliegenden Wellenkämmen (103a) umfaßt.

## Claims

1. A disposable nappy with a skin-contacting cover sheet, which is provided with an elastic opening (16), wherein the nappy (10) comprises a first cover sheet (11) permeable to liquids, an outer sheet (12) impermeable to liquids, a fluid absorbing core (13) which is disposed between the first cover sheet (11) and the outer sheet (12) and a liquid-resistant second cover sheet (14) which lies over the first cover sheet (11) in order to form the skin-contacting cover sheet, the second cover sheet (14) being provided substantially in its central region with the opening (16) extending further in the longitudinal direction than in the transverse direction of the second cover sheet (14), the second cover sheet (14) being connected along its outer periphery to the first cover sheet (11) and elements (17a, 17b) which are elastically extensible in the longitudinal direction being fitted on the second cover sheet (14) along the mutually opposite side edges of the opening (16), wherein the elastic elements (17a, 17b) comprise first and second elastic elements (17a, 17b) which are provided independently of one another in such a way that they extend along the mutually opposite side edges of the opening (16), wherein the first and second elastic elements (17a, 17b) are arranged beyond the ends of the opening (16) opposite one another in the longitudinal direction on the side edges of the second cover sheet (14) and terminate substantially at the ends of the second cover sheet (14) opposite one another in the longitudinal direction.

2. A nappy according to claim 1, wherein the second cover sheet (14) comprises first and second sheet elements (14a, 14b).

3. A nappy according to claim 1, wherein the first and second layer elements (14a, 14b) are connected together along inner side edges opposite one another on both sides in the vicinity of ends of the first and second layer elements (14a, 14b) opposite one another in the longitudinal direction.

4. A nappy according to claim 1, wherein the first and second elastic elements (17a, 17b) intersect or contact one another at the ends of the opening (16) opposite one another in the longitudinal direction.

5. A method of forming a skin-contacting cover sheet of a disposable nappy with an elastic opening (16), with the following steps:
A. feeding first and second elastic elements (17a, 17b), which are both kept stretched, on to a first continuous band (114a) in the longitudinal direction thereof, so that the first and second continuous elastic elements (17a, 17b) extend on the central region of the first continuous band (114a), which runs spaced from first and second side edges opposite one another in the transverse direction, the first and second continuous elastic elements (17a, 17b) being spaced from one another in the transverse direction of the first continuous band (114a), wherein they describe alternate wave crests (103a) and relatively wide wave troughs (103b), and wherein the wave crests (103a) which are described by the first elastic element (17a) are arranged opposite central regions of the respective wave troughs (103b) which are described by the second continuous elastic element (17b) and the wave crests (103a) which are described by the second elastic element (17b) are arranged opposite central regions of the respective wave troughs (103b) which are described by the first continuous elastic element (17a), and simultaneous bonding of the first and second elastic elements (17a, 17b) using an adhesive, in order to form a first continuous composite band (114b);
B. bonding a second continuous band (114c) to the first continuous composite band (114b) in the region thereof within which the first and second continuous elastic elements (17a, 17b) are fixed to the first continuous composite band (114b), in order to form a second continuous composite band (114d);
C. cutting out the part (114d₁) of the second continuous composite band (114d) which is defined between the first continuous elastic element (17a) and the second continuous elastic element (17b) which extend parallel to one another and are spaced from one another in the transverse direction of the second continuous composite band along the respective wave crests (103a) and wave troughs (103b), in order to form cut-outs which are adapted to bound the opening (16) and thus to form third continuous composite band elements (A) (114e₁) and (B) (114e₂);
D. displacing the third continuous composite band elements (A) (114e₁) and (B) (114e₂) relative to one another in the longitudinal direction, so that the respective wave crests (103a) of the third continuous composite band elements (A) (114e₁) and (B) (114e₂) lie opposite one another symmetrically and in overlapping relationship in the transverse direction of these third continuous composite band elements (A) (114e₁) and (B) (114e₂) and the wave troughs (103b) thereof are also arranged symmetrically opposite one another but form the respective openings (16) therebetween; and
E. cutting the third continuous composite band elements (A) (114e₁) and (B) (114e₂) in the transverse direction thereof along conceptual lines (104) which divide vertically in halves the respective pairs of wave crests (103a) arranged on the two sides opposite one another.

6. A method according to claim 5, wherein step D comprises a step of bonding the third continuous composite band elements (A) (114e₁) and (B) (114e₂) together in overlapping relationship along their inner side edges of the respective pairs of wave crests (103a) opposite one another on the two sides.

## Revendications

1. Lange jetable comportant une couche de recouvrement en contact avec la peau, pourvue d'une ouverture élastique (16), le lange (10) présentant une première couche de recouvrement perméable aux liquides (11), une couche extérieure imperméable aux liquides (12), un corps central absorbant les liquides (13), emprisonné entre la première couche de recouvrement (11) et la couche extérieure (12), et une seconde couche de recouvrement(14) à l'épreuve des liquides, posée sur la première couche de recouvrement (11), pour former la couche de recouvrement en contact avec la peau, la seconde couche de recouvrement (14) étant pourvue, pour l'essentiel en son centre, d'une ouverture (16) plus large dans le sens longitudinal de ladite seconde couche de recouvrement (14) que dans le sens transversal de cette dernière, la seconde couche de recouvrement (14) étant jointe a la première (11) sur son pourtour extérieur, et des éléments élastiques (17a, 17b), étirables dans le sens longitudinal, étant appliqués sur la seconde couche de recouvrement (14) le long des bords latéraux mutuellement opposés de l'ouverture (16), lange dans lequel les éléments élastiques (17a, 17b) sont constitués par un premier et un second élément élastique (17a, 17b), disposés indépendamment l'un de l'autre de façon à ce qu'ils longent les bords latéraux mutuellement opposés de l'ouverture (16), ces premier et second éléments élastiques étant appliqués au-delà des extrémités de l'ouverture (16) opposées dans le sens longitudinal, sur les bords latéraux de la seconde couche de voilage (14), et se terminent, sensiblement, aux extrémités de la seconde couche de voilage (14) opposées dans le sens de la longueur.

2. Lange selon la revendication 1, dans lequel la seconde couche de recouvrement (14) comprend un premier et un second pans (14a, 14b).

3. Lange selon la revendication 1, dans lequel les premier et second pans sont raccordés entre eux le long de bords latéraux intérieurs en opposition mutuelle des deux côtés, à proximité des extrémités des premier et second pans (14a, 14b) opposées dans le sens de la longueur.

4. Lange selon la revendication 1, dans lequel les premier et second éléments élastiques (14a, 14b) se chevauchent ou se touchent aux extrémités de l'ouverture (16) longitudinalement opposées.

5. Procédé pour ménager une ouverture élastique (16) dans une couche de voilage d'un lange jetable, en contact avec la peau, comprenant les opérations suivantes :
A. - présentation d'un premier et d'un second élément élastique (17a, 17b), tous deux maintenus à l'état étiré, sur une première bande continue (114a), dans le sens de sa longueur, en sorte que les premier et second éléments élastiques continus (17a, 17b) se situent dans la zone médiane de la première bande continue (114a), zone écartée d'un premier et d'un second bord latéral en opposition transversale mutuelle, les premier et second éléments élastiques (17a, 17b) continus étant écartés l'un de l'autre transversalement à la bande continue (114a), en décrivant, en alternance, des crêtes d'ondulations (103a) et des creux d'ondulations (103b) relativement larges, les crêtes d'ondulations respectives (103a), dessinées par le premier élément élastique (17a), faisant face à des zones médianes des creux d'ondulations (103b) décrits par le second élément élastique continu (17b), et les crêtes d'ondulations (103a), dessinées par le second élément élastique continu (17b), faisant face à des zones médianes des creux d'ondulations (103b) décrits par le premier élément élastique continu (17a), et collage simultané du premier et du second élément élastique (17a, 17b), avec un adhésif, pour former une première bande continue composée (114b);
B. - jonction d'une deuxième bande continue (114c) avec la première bande continue composée (114b), dans la zone de cette dernière où les premier et second éléments élastiques continus (17a, 17b) sont fixés à la première bande continue composée (114b), de façon à former une deuxième bande continue composée (114d);
C. - suppression, par découpage, de la partie (114d₁) de la deuxième bande continue composée (114d) qui est définie entre le premier élément élastique continu (17a) et le second élément élastique continu (17b), lesquels s'étendent parallèlement entre eux et s'écartent l'un de l'autre transversalement à la deuxième bande continue composée (114d), en longeant les crêtes (103a) et les creux d'ondulations (103b), pour former ainsi des découpures (16a) destinées à délimiter l'ouverture (16), et pour former de cette manière des troisièmes éléments de bande continue composée (A) (114e₁) et (B) (114e₂);
D. - glissement des troisièmes éléments de bande continue composée (A) (114e₁) et (B) (114e₂) l'un part rapport à l'autre, dans le sens longitudinal, de façon à ce que les crêtes d'ondulations (103a) des troisièmes éléments de bande continue composée (A) (114e₁) et (B) (114e₂) viennent se positionner face à face, symétriquement l'un à l'autre, en se chevauchant, transversalement à ces troisièmes éléments de bande continue composée (A) (114e₁) et (B) (114e₂), et à ce que les creux d'ondulations (103b) de ceux-ci soient également positionnés face à face, symétriquement les uns aux autres, mais forment, entre eux, les ouvertures (16) respectives; et
E. - découpage des troisièmes éléments de bande continue composée (A) (114e₁) et (B) (114e₂), transversalement à ceux-ci, en suivant des lignes fictives (104) partageant verticalement en deux moitiés les deux crêtes d'ondulations (103a) se faisant face, de part et d'autre desdites lignes.

6. Procédé selon la revendication 5, dans lequel l'étape D comprend une opération de jonction des troisièmes éléments de bande continue composée (A) (114e₁) et (B) (114e₂), en les faisant se chevaucher suivant des bords latéraux intérieurs de chaque paire de crêtes d'ondulations (103a) en vis-à-vis.
